# EUROPÄISCHE PATENTSCHRIFT

(11) **EP 0 882 140 B1**
(45) Veröffentlichungstag und Bekanntmachung des Hinweises auf die Patenterteilung: **28.08.2002**
(21) Anmeldenummer: 97919250.7
(22) Anmeldetag: 14.02.1997
(51) Int. Cl.: C12Q 1/68

(54) **VERFAHREN ZUR ERKENNUNG DES ERKRANKUNGSRISIKOS NACH ARTHROPODENSTICH**
PROCESS FOR RECOGNIZING THE RISK OF BECOMING ILL AFTER AN ARTHROPOD BITE
PROCEDE POUR EVALUER LES RISQUES DE DEVELOPPEMENT D'UNE MALADIE APRES UNE PIQURE PAR UN ARTHROPODE

(30) Priorität: 14.02.1996 DE 19605475
(43) Veröffentlichungstag der Anmeldung: 09.12.1998
(73) Patentinhaber: Von Knebel Doeberitz, Magnus, Prof. Dr., 69118 Heidelberg (DE); Maiwald, Matthias, Palo Alto, CA 94304 (US)
(72) Erfinder: Von Knebel Doeberitz, Magnus, Prof. Dr., 69118 Heidelberg (DE); Maiwald, Matthias, Palo Alto, CA 94304 (US)
(74) Vertreter: Schüssler, Andrea, Dr.
(86) Internationale Anmeldenummer: DE9700311
(87) Internationale Veröffentlichungsnummer: WO97030177

(56) Entgegenhaltungen:
- EP-A- 0 421 725
- SHOTT S R: "Diagnosis and management of Lyme Disease" CURRENT OPINION IN OTOLARYNGOLOGY AND HEAD AND NECK SURGERY, Bd. 3, Nr. 6, Dezember 1995, Seiten 379-382, XP002042421
- PEAVEY C: "Transmission of Borellia Burgdorferi by Ixodes Pacificus" JOURNAL OF PARISITOLOGY, Bd. 81, Nr. 2, April 1995, Seiten 175-8, XP002042422
- SCHWARTZ J ET AL: "Diagnosis of early Lyme Disease by polymerase chain reaction amplification and culture of skin biopsies from Erythema Migrans lesions" J. CLIN. MICROBIOL, Bd. 30, Dezember 1992, Seiten 3082-88, XP002042423 in der Anmeldung erwähnt

## Beschreibung

Die vorliegende Erfindung betrifft den Nachweis einer Nukleinsäure eines durch einen Arthropoden gestochenen Wirts und einer Nukleinsäure eines durch den Arthropoden übertragenen Krankheitserregers im Arthropoden. Ferner betrifft die Erfindung einen zur Durchführung des Nachweises geeigneten Kit.

Es gibt weltweit eine Vielzahl von Arthropoden, die menschliche oder tierische Wirte durch Stich oder Biß befallen. Diese Arthropoden sind Überträger einer Vielzahl von Krankheitserregern, z.B. Viren, Bakterien, Protozoen oder Würmern. Der bekannteste von Arthropoden, insbesondere Zecken, übertragene Krankheitserreger ist Borrelia burgdorferi, der Lyme-Borreliose hervorruft. Im Nachfolgenden werden die Zusammenhänge und Abläufe bei einem Arthropoden-, insbesondere Zeckenstich, am Beispiel der Lyme-Borreliose erklärt.

Die Lyme-Borreliose ist eine Erkrankung, welche durch Zeckenstich übertragen wird und in weiten Teilen der nördlichen Hemisphäre vorkommt. In Mitteleuropa und in Nordamerika ist es die häufigste durch Arthropoden übertragene Erkrankung. Die Befallsrate von Zecken mit dem Erreger Borrelia burgdorferi zeigt regionale Schwankungen. Aus den USA werden Befallsraten von bis zu 55% berichtet, während für Europa Berichte über Befallszahlen zwischen 2,5% und 36% existieren.

Der Krankheitsverlauf der Lyme-Borreliose ist stadienhaft. Nach Zeckenstich erfolgt mit einer Inkubationszeit von mehreren Tagen bis wenigen Wochen zunächst eine Lokalinfektion (Stadium I; Erythema migrans), im weiteren Verlauf eine Erregergeneralisation (Stadium II; Symptome am Nervensystem, am Herzen oder an Gelenken) und nach Monaten oder Jahren eine Organmanifestation (Stadium III, spätes Stadium; Lyme-Arthritis oder Acrodermatitis chronica atrophicans). Einzelne Erkrankunsstadien können auch übersprungen werden oder es kann zur spontanen Ausheilung kommen.

Insgesamt ist sowohl die klinische als auch die Labordiagnostik der Lyme-Borreliose schwierig und in einigen Krankheitsfällen mit großer Unsicherheit behaftet. Da der direkte Nachweis des Erregers in Materialien von Patienten als unzuverlässig gilt, werden meist serologische Verfahren als Hilfestellung für die Diagnose verwendet. In frühen Krankheitsstadien werden jedoch häufig nicht erhöhte Antikörpertiter gefunden, obwohl eine Infektion stattgefunden hat, so daß hier die Serologie bisweilen kein verläßliches Kriterium darstellt. Dies trifft teilweise sogar auf die Neuroborreliose zu, die ein bedrohliches Krankheitsbild darstellen kann und deren Abgrenzung von neurologischen Erkrankungen anderer Ursache von großer Bedeutung ist.

Die dem Stand des Wissens entsprechende Therapie der Lyme-Borreliose erfolgt ab dem Stadium II der Erkrankung durch intravenöse Verabreichung von Antibiotika. Eine solche Verabreichung erfordert entweder tägliche Arztbesuche oder die stationäre Aufnahme des Patienten im Krankenhaus. Allein die Kosten für die verabreichten Antibiotika betragen derzeit etwa 2500,- bis 5000,- DM pro Therapiezyklus. Teilweise werden sehr aggressive Krankheitsverläufe beobachtet, die mehrere Therapiezyklen erfordern und damit sehr hohe Behandlungskosten verursachen.

Angaben über das Infektionsrisiko nach Zeckenstich sind bislang spärlich und meist ohne Bezug zur Positivität oder Negativität der Zecke ermittelt worden. Amerikanische Untersuchungen gehen davon aus, daß 1-3% der Zeckenstiche in Endemiegebieten zur Infektion mit Borrelia burgdorferi führt. Dabei kommen Magid et al., N. Engl. J. Med. 327, (1992), 534-541 in einer statistischen Kosten-Nutzen-Analyse zu dem Schluß, daß es in Gebieten mit sehr hoher Durchseuchung der Zecken mit Borrelia burgdorferi sinnvoll sei, nach jedem Zeckenstich prophylaktisch Antibiotika zu verabreichen. In Deutschland wird derzeit eine Antibiotikaprophylaxe nach Zeckenstich abgelehnt. Anstelle dessen wird empfohlen, erst beim Auftreten von Symptomen einer Lyme-Borreliose gezielt eine Therapie einzuleiten. Diese Empfehlung besitzt zwei wesentliche Nachteile: (1) Einzelne Stadien der Erkrankung (z. B. Erythema migrans) können übersprungen werden und die Erkrankung kann mit den schwierig zu diagnostizierenden und teuer zu therapierenden Symptomen des Stadium II oder III beginnen. (2) Wenn es eine zuverlässige Möglichkeit gibt, früh nach Zeckenstich das Infektionsrisiko abzuschätzen, ist es unter ethischen Gesichtspunkten kaum zu rechtfertigen, Patienten dem Risiko einer Erkrankung und den damit verbundenen Leiden auszusetzen. Zu diesen Nachteilen kommt hinzu, daß eine Antibiotikaprophylaxe oder die Therapie einer frühen Infektion wesentlich einfacher und kostengünstiger durchzuführen ist als die Therapie einer Infektion zum späteren Zeitpunkt.

Bislang gibt es kaum Kriterien, das Erkrankungsrisiko für einen individuellen Wirt nach individuellem Arthropodenstich oder -biß zuverlässig abschätzen zu können und daraus eine Indikation zur prophylaktischen Behandlung ableiten zu können. Bei der Lyme-Borreliose sind die bisherigen Empfehlungen zum Verhalten nach Zeckenstich vor dem Hintergrund entstanden, daß konventionelle Nachweisverfahren für Borrelia burgdorferi in Zecken, wie Kultur, Dunkelfeldmikroskopie oder direkte Immunfluoreszenz, keine ausreichende Zuverlässigkeit besitzen. Ursache dafür ist einerseits die geringe Sensitivität der Verfahren und andererseits, daß Blutbestandteile in Zecken, die bereits mit dem Saugvorgang begonnen haben, diese Nachweisverfahren in erheblichem Maße stören. Somit war es bislang mit konventionellen Methoden nicht mit ausreichender Sicherheit möglich, das aus einem Zeckenstich für den betroffenen Menschen resultierende Infektionsrisiko festzustellen.

Die Aufgabe der vorliegenden Erfindung bestand deshalb darin, ein Verfahren bereitzustellen, mit dem es möglich ist, direkt nach Arthropodenstich bzw. -biß beim betroffenen Wirt oder Patienten eine möglichst genaue Abschätzung des Risikos vornehmen zu können, ob dieser eine durch Arthropoden übertragene Erkrankung bekommen wird und in Abhängigkeit von der ermittelten Höhe des Risikos eine Prophylaxe, insbesondere mit Antibiotika, vornehmen zu können.

Erfindungsgemäß wird dies durch ein Verfahren gemäß Anspruch 1 erreicht. Vorteilhafte Ausgestaltungen wie auch einen zur Durchführung des Verfahrens geeigneten Kit ergeben sich aus den Unteransprüchen.

Das erfindungsgemäße Verfahren beruht auf der Erkenntnis der Anmelderin, daß im Rahmen eines Stichs oder Bisses durch einen Arthropoden zum einen Bestandteile des Wirts vom Arthropoden aufgenommen werden, und zum anderen Bestandteile vom Arthropoden an den Wirt abgegeben werden. Unter den vom Arthropoden an den Wirt abgegebenen Bestandteilen können auch die erwähnten Krankheitserreger sein. So kann man durch Untersuchung des Arthropoden darauf schließen, ob dieser Bestandteile des Wirts aufgenommen hat und gleichzeitig Krankheitserreger beherbergt.

Der Nachweis einer Nukieinsäure des Wirts und solcher eines durch den Arthropoden übertragenen Krankheitserregers im Arthropoden erfolgt durch übliche Verfahren, vorzugsweise mittels PCR. Für eine PCR wird der Arthropode vom Wirt entfernt und Nukleinsäure aus dem Arthropoden isoliert. Diese wird unter Verwendung geeigneter Primer für die Wirts-Nukleinsäure bzw. solcher für die Nukleinsäure eines Krankheitserregers amplifiziert und die Amplifikationsprodukte werden nachgewiesen, wobei die einzelnen Schritte getrennt oder gemeinsam erfolgen können. Mittels dieser Diagnostik kann das Infektionsrisiko für den Wirt sicher vorausgesagt werden.

Der Ausdruck "Nukleinsäure" umfaßt RNA und DNA, der Ausdruck "Wirt" Tiere und Menschen. Ferner umfaßt der Ausdruck "Arthropode" Insekten, insbesondere Zecken und Mücken, Spinnen und Krebse. Desweiteren umfaßt der Ausdruck "Krankheitserreger" Viren, Bakterien, Protozen und Würmer.

Durch den Nachweis sowohl von Nukleinsäure-Sequenzen des Wirts als auch von erregerspezifischen Nukleinsäure-Sequenzen im Arthropoden, gegebenenfalls in Kombination, ist eine wesentlich genauere Abschätzung des Erkrankungsrisikos nach Arthropodenstich oder -biß möglich als dies bei Anwendung konventioneller Erreger-Nachweisverfahren, wie Kultur oder Mikroskopie, oder auch beim alleinigen Nachweis von Nukleinsäure des Erregers im Wirt der Fall wäre.

Nachstehend wird das erfindungsgemäße Verfahren beispielhaft zum Nachweis von Zecken beschrieben, die Borrelia burgdorferi, den Erreger der Lyme-Borreliose tragen. Dieser Erreger liegt im Mitteldarm der Zecken, nicht jedoch oder nur selten im Speichel dieser vor. In einer ersten Phase nach Zeckenstich wird Zeckenspeichel in die Stichwunde eingebracht, welcher anästhesierende und gewebsverflüssigende Eigenschaften besitzt. In einer zweiten Phase werden Blut und Gewebsbestandteile angesaugt. In dieser Phase kommt es oftmals zur Regurgitation von Mitteldarminhalt, welcher Krankheitserreger, insbesondere Borrelien, enthalten kann, in die Stichwunde. Somit folgt die Übertragung des Erregers erst nach einem Ansaugen menschlicher Zellbestandteile, so daß bei der Anwesenheit von menschlicher Nukleinsäure in der Zecke von einem höheren Übertragungsrisiko ausgegangen werden kann. Diese Beziehung wird untermauert durch tierexperimentelle Untersuchungen, die eine klare Beziehung zwischen Saugdauer der Zecken und Übertragungshäufigkeit des Erregers zeigen. Nur wenn die Zecke lang genug Blut ansaugen kann, findet sich dieses auch im Zeckenkörper wieder und eine lange Aufenthaltsdauer der Zecke am Wirt vergrößert auch das Risiko, daß es zur Übertragung von Krankheitserregern kommt.

Nach Gewinnung von Nukleinsäure, insbesondere DNA, aus einem von einem Wirt entfernten Arthropoden, insbesondere einer Zecke, wird diese in einer PCR-Reaktion eingesetzt. Dabei besteht die Möglichkeit, in einer ersten PCR-Reaktion zuerst ein Primersystem für die vom Arthropoden übertragenen Krankheitserreger, insbesondere Borrelia burgdorferi, zu verwenden und in einer nachfolgenden PCR-Reaktion ein Primersystem für ein wirtsspezifisches Gen, z.B. für ein menschliches Gen, insbesondere c-myc-Onkogen oder Aktingen, zu verwenden. Es besteht allerdings auch die Möglichkeit, die Amplifikation in einer einzigen PCR-Reaktion durchzuführen. Die Durchführung der PCR-Reaktion erfolgt anhand der dem Fachmann bekannten Standardbedingungen.

Als Primersystem für Borrelia burgdorferi-DNA kommt beispielsweise in Frage: JS1 (5'-AGAAGTGCTGGAGTCGA-3'), JS2 (5'-TAGTGCTCTACCTCTATTAA-3') und FS1 (5'-AGTCTGTTTAAAAAGGCA) (Schwartz, J. et al., J. Clin. Microbiol. 30, (1992), 3082-3088).

Als Primersystem für die DNA eines menschlichen c-myc-Onkogens kommt beispielsweise in Frage:
o1 (5'-CTGGTTTTCCACTACCCGAA-3'), o2 (5'-CCGCAACCCTTGCCGCATCC-3') und o3 (5'-GACGCGGGGAGGCTATTCTG-3') (Wolf, J. et al., Cancer Res. 50, (1990), 3095-3100).

Nach Beendigung der PCR-Reaktion wird die durch PCR amplifizierte DNA auf z.B. einem 5-10% igen Polyacrylamidgel aufgetrennt und auf eine Membran, z.B. Nylonmembran, übertragen. Nach Fixierung der DNA auf der Membran wird diese in einer oder mehreren Hybrisierungsreaktionen mit radioaktiv markierten Fragmenten, die entweder für einen vom Arthropoden übertragenen Krankheitserreger, insbesondere Borrelia burgdorferi, spezifisch sind oder für den Wirt (Patient) spezifisch sind, eingesetzt. Nach Auswertung eines aufgelegten Röntgenfilms kann für den Wirt (Patient) entschieden werden, ob der Arthropode, der ihn gestochen hat, menschliche DNA als auch DNA eines Krankheitserregers enthalten hat. im Fall, daß in dem Arthropoden beide Sequenzen nachweisbar waren, sollte prophylaktisch eine Antibiotikabehandlung begonnen werden. Dies bedeutet, daß nur die Patienten behandelt werden brauchen, für die ein Erkrankungsrisiko besteht und nicht alle, die einen Anthropodenstich haben. Dies stellt einen großen Vorteil hinsichtlich Kosten und Belastung für den Patienten dar.

Erfindungsgemäß wird auch ein Kit zur Durchführung vorstehender Verfahren bereitgestellt. Insbesondere handelt es sich um einen Kit für ein PCR-Verfahren, wobei der Kit vorzugsweise Primer für menschliche Nukleinsäure, insbesondere DNA, ganz besonders für die DNA eines c-myc-Onkogens, und Primer für Borrelia burgdorferi Nukleinsäure, insbesonders DNA, sowie für die Durchführung einer PCR geeignete Puffer, Enzyme und Trägerstoffe enthält. Ganz besonders bevorzugt enthält der Kit vorstehend genannte Primersysteme.
Die Erfindung wird nachfolgend anhand eines Beispiels näher erläutert.

### Beispiel: Nachweis von Borrelia burgdorferi DNA und von menschlicher DNA in Zecken

Es wurden 22 Zecken untersucht, die Menschen gestochen hatten. Zum Nachweis von Borrelia burgdorferi-DNA und von menschlicher DNA in Zecken wurde eine Polymerase-Kettenreaktion unter Verwendung eines Primersystems für Borrelia burgdorferi und eines Primersystems für das menschliche c-*myc*-Onkogen durchgeführt. Die Oligonukleotide für Borrelia burgdorferi waren: JS1 (5'-AGAAGTGCTGGAGTCGA-3'), JS2 (5'-TAGTGCTCTACCTCTATTAA-3') und FS1 (5'-AGTCTGTTTAAAAAGGCA-3'). Diejenigen für das c-*myc*-Onkogen waren: o1 (5'-CTGGTTTTCCACTACCCGAA-3'), o2 (5'-CCGCAACCCTTGCCGC-ATCC-3') und o3 (5'-GACGCGGGGAGGCTATTCTG-3'). Dabei wurden die bereits für Urinproben von Patienten publizierten Bedingungen für die Amplifikation mittels PCR und den Nachweis der PCR-Produkte angewandt (vgl. Maiwald et al., BIOforum 17, (1994), 232-237).

Ein PCR-Ansatz enthielt in 100 µl 50 mM KCI, 50 mM Tris (pH 9), 2,0 mM MgCl₂, 200 µM dNTPs (Desoxynukleosid-Triphosphate), 500 nM jedes Primers und 2,5 Einheiten *Taq*-Polymerase (AmpliTaq® DNA Polymerase, Perkin-Elmer, Norwaik, CT, USA), sowie 70 µl Mineralöl. Als Probevolumen wurde 10 µl gewählt. Die Aufbereitung der Zecken für die PCR erfolgte, indem diese mit einem Skalpell zerteilt wurden, in ein 1,5 ml-Reaktionsgefäß überführt wurden, dort mit Hilfe eines Pistills (Kontres Instruments, USA) zerdrückt und dann mit 30 µl einer 20%igen Chelex-Suspension (Bio-Rad, USA) für 10 min aufgekocht wurden. Vom Überstand des Chelex-Harzes wurden 10 µl in die PCR-Reaktion eingesetzt.

Zum Nachweis der PCR-Produkte wurden 30 µl aus den PCR-Reaktionsansätzen mittels Elektrophorese auf 8 %-igen Polyacrylamidgelen (mit TBE-Puffer; 0.089 M Tris, 0.089 M Borsäure, 0.002 M EDTA) bei 12 V cm⁻¹ für 2-3 h aufgetrennt und anschließend wurden die Gele mit Ethidiumbromid angefärbt und auf einem UV-Transilluminator photographiert. Für die anschließende Hybridisierung wurde die DNA im Gel für 15 min denaturiert (0.2 M NaOH, 0.6 M NaCl), und danach für 2 x 15 min in 25 mM Natriumphosphatpuffer (pH 7,0) neutralisiert. Die DNA aus dem Gel wurde dann auf eine Nylon-Membran (Sartolon, 0.2 µm Porengröße, Fa. Sartorius) mittels Elektroblot in TBE-Puffer bei 110 mA über Nacht transferiert.

Nach Hitzefixierung der DNA auf der Membran (80° C, 2 h) wurde die Membran bei 47 °C für mindestens 2 h prähybridisiert (in 0.9 M NaCI, 6 mM EDTA, 90 mM Tris, 1% SDS mit 1 g/l Hefe-RNA) und anschließend über Nacht bei 47° C mit dem durch Kinasereaktion (Sambrook et al., Molecular cloning: A laboratory manual. 2nd ed., Cold Spring Harbor Laboratory Press, Cold Spring Harbor, N. Y., (1989)) mit ³²P markierten Oligonukleotid FS1 (spezifisch für Borrelia burgdorferi; vgl. vorstehend) in 10 ml der Prähybridisierungslösung hybridisiert. Die Nylonmembran wurde anschließend in einer Lösung aus 3 x SSC (20 x SSC ist 3 M NaCl, 300 mM Natriumcitrat, pH 7.2), 5% SDS and 10 mM Natriumphosphat (pH 7.0) bei 47° C für 10 min gewaschen, dann noch einmal in 1 x SSC, 1% SDS bei 47° C für 10 min gewaschen, und anschließend mit einem Röntgenfilm (X-OMAT AR, Kodak, USA) in einer Kassette exponiert.

Es wurden sechs Zecken identifiziert, die Borrelia burgdorferi-DNA und menschliche DNA aufwiesen. Von den entsprechenden sechs Patienten entwickelten drei nach klinischen und serologischen Kriterien eine Lyme-Borreliose. Somit erweist sich der Nachweis von Borrelia burgdorferi-DNA zusammen mit menschlicher DNA in Zecken als geeignet, den durch eine Infektion gefährdeten Personenkreis einzugrenzen.

Ein weiteres Kollektiv von 71 Zecken, die an Patienten gesaugt hatten und deren Saugdauer protokolliert worden war, wurden auf die Anwesenheit des menschlichen c-*myc*-Gens mittels des oben beschriebenen PCR-Verfahrens untersucht. Für die Hybridisierungsreaktion wurde das ³²P markierte Oligonukleotid o3 (spezifisch für menschlisches c-myc-Onkogen; vgl. vorstehend) als Hybridisierungsprobe eingesetzt. In Abhängigkeit von der Saugdauer zeigten sich folgende Ergebnisse: (1) 0-4 h Saugdauer: 3 von 13 Zecken positiv (d.h. es konnte menschliche DNA in der Zecke nachgewiesen werden); (2) 5-12 h: 2 von 11 Zecken positiv; (3) 13-24 h: 2 von 18 Zecken positiv; (4) 25-48 h: 8 von 19 Zecken positiv; (5) länger als 48 h: 3 von 5 Zecken positiv. Somit konnte gezeigt werden, daß mit zunehmender Saugdauer der Zecken die Wahrscheinlichkeit der Anwesenheit menschlicher DNA in Zecken statistisch signifikant zunimmt.

## Patentansprüche

1. Verfahren zur Erkennung des Erkrankungsrisikos nach Arthropodenstich, **dadurch gekennzeichnet, daß** nach Stich oder Biß eines Wirts durch einen Arthropoden in letzterem das Vorliegen von Nukleinsäure des Wirts und von Nukleinsäure eines durch den Arthropoden übertragenen Krankheitserregers nachgewiesen wird.

2. Verfahren nach Anspruch 1, **dadurch gekennzeichnet, daß** die Nukleinsäure DNA ist.

3. Verfahren nach Anspruch 1 oder 2, **dadurch gekennzeichnet, daß** der Nachweis der Wirts-Nukleinsäure und solcher des Krankheitserregers mittels PCR erfolgt.

4. Verfahren nach einem der Ansprüche 1 - 3, **dadurch gekennzeichnet, daß** es sich bei dem Arthropoden um eine Zecke oder eine Mücke handelt.

5. Verfahren nach einem der Ansprüche 1 - 4, **dadurch gekennzeichnet, daß** es sich bei dem von einem Arthropoden übertragenen Krankheitserregers um Viren, Bakterien, Protozoen oder Würmer handelt.

6. Verfahren nach einem der Ansprüche 1 - 5, **dadurch gekennzeichnet, daß** es sich bei dem Krankheitserreger um Borrelia burgdorferi handelt.

7. Verfahren nach einem der Ansprüche 1 - 6, **dadurch gekennzeichnet, daß** es sich bei dem Wirt um einen Menschen handelt.

8. Verfahren nach Anspruch 7, **dadurch gekennzeichnet, daß** in der PCR bezüglich der Wirts-DNA Primer für menschliches C-myc-Onkogen verwendet werden.

9. Verfahren nach Anspruch 8, **dadurch gekennzeichnet, daß** die Primer folgende Sequenz haben: o1: 5'-CTGGTTTTCCACTACCCGAA-3', o2: 5'-CCGCAACCCTTGCCGCATCC und/oder

o3: 5'-GACGCGGGAGGCTATTCTG-3'.

10. Verfahren nach Anspruch 6, **dadurch gekennzeichnet, daß** in der PCR bezüglich der DNA des Krankheitserregers Primer für Borrelia burgdorferi verwendet werden.

11. Verfahren nach Anspruch 10, **dadurch gekennzeichnet, daß** die Primer folgende Sequenz haben:

JS1: 5'-AGAAGTGCTGGAGTCGA-3', JS2:
5'-T A G T G C T C T A C C T C T A T T A A - 3' u n d / o d e r F S 1 :
5'-AGTCTGTTTAAAAAGGCA-3'.

12. Kit zur Durchführung des Verfahren nach einem der Ansprüche 2-11, umfassend Primer für menschliche DNA, insbesondere für C-myc-Onkogen, und solche für Borrelia burgdorferi DNA sowie zur Durchführung einer PCR notwendigen Puffer, Enzyme und Trägerstoffe.

## Claims

1. A process for recognizing the risk of falling ill after an arthropod bite, **characterized in that** after the prick or bite of a host by an arthropod, nucleic acid of the host and nucleic acid of a pathogen passed along or on by the arthropod are demonstrated to be present in the latter.

2. The process according to claim 1, **characterized in that** the nucleic acid is DNA.

3. The process according to claim 1 or 2, **characterized in that** the nucleic acid from the host and that of the pathogen are detected by means of PCR.

4. The process according to any one of claims 1 to 3, **characterized in that** the arthropod is a tick or mosquito.

5. The process according to any one of claims 1 to 4, **characterized in that** the arthropod-borne pathogens are viruses, bacteria, protozoa or worms.

6. The process according to any one of claims 1 to 5, **characterized in that** the pathogen is Borrelia burgdorferi.

7. The process according to any one of claims 1 to 6, **characterized in that** the host is a human being.

8. The process according to claim 7, **characterized in that** PCR uses primers for human c-myc oncogene with respect to the host DNA.

9. The process according to claim 8, **characterized in that** the primers have the following sequences: o1:  5'- CTGGTTTTCCACTACCCGAA-3', o2: 5'-CCGCAACCCTTGCCGCATCC, and/or

o3: 5'-GACGCGGGAGGCTATTCTG-3'.

10. The process according to claim 6, **characterized in that** the PCR uses primers for Borrelia burgdorferi with respect to the DNA of the pathogen.

11. The process according to claim 10, **characterized in that** the primers have the following sequences: JS1: 5'-AGAAGTGCTGGAGTCGA-3', JS2: 5'-TAGTGCTCTACCTCTATTAA-3', and /or

FS1: 5'-AGTCTGTTTAAAAAGGCA-3'.

12. A kit for carrying out the process according to any one of claims 2 to 11, comprising primers for human DNA, particularly for c-myc oncogene, and those for Borrelia burgdorferi DNA as well as buffers, enzymes and carriers, all necessary to carry out a PCR.

## Revendications

1. Procédé pour identifier le risque de contamination après piqûre par un arthropode, **caractérisé en ce qu'**après piqûre ou morsure d'un hôte par un arthropode, la présence chez ce dernier d'acide nucléique de l'hôte et d'acide nucléique d'un agent pathogène transmis par l'arthropode est détectée.

2. Procédé suivant la revendication 1, **caractérisé en ce que** l'acide nucléique est un ADN.

3. Procédé suivant la revendication 1 ou 2, **caractérisé en ce que** la détection de l'acide nucléique de l'hôte et de celui de l'agent pathogène est effectuée par réaction en chaîne de la polymérase.

4. Procédé suivant l'une des revendications 1 à 3, **caractérisé en ce que** l'arthropode est un moustique ou une tique.

5. Procédé suivant l'une des revendications 1 à 4, **caractérisé en ce que** les agents pathogènes transmis par un arthropode sont des virus, des bactéries, des protozoaires ou des vers.

6. Procédé suivant l'une des revendications 1 à 5, **caractérisé en ce que** l'agent pathogène est *Borrelia burgdorferi.*

7. Procédé suivant l'une des revendications 1 à 6, **caractérisé en ce que** l'hôte est un être humain.

8. Procédé suivant la revendication 7, **caractérisé en ce qu'**on utilise dans la réaction en chaîne de la polymérase, en ce qui concerne l'ADN de l'hôte, des amorces pour l'oncogène C-myc humain.

9. Procédé suivant la revendication 8, **caractérisé en ce que** les amorces ont la séquence suivante : o1: 5'-CTGGTTTTCCACTACCCGAA-3', o2: 5'-CCGCAACCCTTGCCGCATCC und/oder

o3: 5'-GACGCGGGGAGGCTATTCTG-3'.

10. Procédé suivant la revendication 6, **caractérisé en ce qu'**on utilise dans la réaction en chaîne de la polymérase, en ce qui concerne l'ADN de l'agent pathogène, des amorces pour *Borrelia burgdorferi*.

11. Procédé suivant la revendication 10, **caractérisé en ce que** les amorces ont la séquence suivante :

JS1: 5'-AGAAGTGCTGGAGTCGA-3', JS2:
5'-TAGTGCTCTACCTCTATTAA-3' und/oder FS1:
5'-AGTCTGTTTAAAAAGGCA-3'.

12. Nécessaire pour la mise en oeuvre du procédé suivant l'une des revendications 2 à 11, comprenant des amorces pour l'ADN humain, en particulier pour l'oncogène C-myc, et des amorces pour l'ADN de *Borrelia burgdorferi*, ainsi que des tampons, des enzymes et des substrats nécessaires pour la conduite de la réaction en chaîne de la polymérase.
